(19) **Europäisches Patentamt**
**European Patent Office**
**Office européen des brevets**

(11) Veröffentlichungsnummer: **0 501 292 A1**

## (12) EUROPÄISCHE PATENTANMELDUNG

(21) Anmeldenummer: **92102729.8**

(22) Anmeldetag: **19.02.92**

(51) Int. Cl.5: **C12N 9/80**, C12N 1/20,
//(C12N9/80,C12R1:01)

(30) Priorität: **28.02.91 DE 4106375**

(43) Veröffentlichungstag der Anmeldung:
**02.09.92 Patentblatt 92/36**

(84) Benannte Vertragsstaaten:
**AT BE CH DE DK ES FR GB GR IT LI LU NL PT SE**

(71) Anmelder: **Degussa Aktiengesellschaft
Weissfrauenstrasse 9
W-6000 Frankturt am Main 1(DE)**

(72) Erfinder: **Kula, Maria-Regina, Prof. Dr.
Selgenbusch 12
W-5162 Niederziehr/Hambach(DE)**
Erfinder: **Joeres, Ulrich
Gatherskamp 101
W-4050 Mönchengladbach(DE)**

(54) **Eine L-Carnitin-Amidase produzierender Mikroorganismus, L-Carnitin-Amidase, Verfahren zu deren Gewinnung und deren Verwendung.**

(57) Beschrieben wird der Mikroorganismus mit der Hinterlegungsnummer DSM 6230, die durch diesen Mikroorganismus produzierte L-Carnitin-Amidase und ein Verfahren zur Gewinnung dieses Enzyms aus dem Mikroorganismus DSM 6230. Sowohl der Mikroorganismus selbst als auch die von ihm produzierte L-Carnitin-Amidase können dazu verwendet werden, selektiv L-Carnitinamid und/oder den L-Anteil von DL-Carnitinamid zu L-Carnitin zu hydrolysieren.

EP 0 501 292 A1

Die Erfindung betrifft den Mikroorganismus mit der Hinterlegungsnummer DSM 6230.

Sie betrifft ferner eine mikrobiologisch hergestellte L-Carnitin-Amidase, welche durch die folgenden Eigenschaften gekennzeichnet ist:

a) Reaktivität:

sie hydrolysiert L-Carnitinamid zu L-Carnitin und Ammoniak,

b) Substratspezifität:

sie hydrolysiert nicht D-Carnitinamid, keine aliphatischen oder aromatischen Carbonsäureamide, keine Aminosäureamide und keine Dipeptidamide,

c) Induktoren:

sie ist ein induzierbares Enzym und wird induziert durch L-Carnitinamid, L-Carnitin, $\gamma$-Butyrobetain und Dehydrocarnitin,

d) pH-Optimum:

der optimale pH-Bereich liegt zwischen pH 7.0 und pH 9,5,

e) pH-Stabilität:

sie zeigt eine gute pH-Stabilität im pH-Bereich zwischen pH 5,0 und pH 9,5,

f) Inhibitoren:

inhibierend wirken bereits bei einer Konzentration von 1 mM $Zn^{2+}$, $Cu^{2+}$, $Hg^{2+}$ und $Ag^{2+}$, ferner p-Hydroxymercuribenzoat, Phenylmethansulfonylfluorid und Dinitrodithiobenzoesäure,

g) Molekulargewicht:

das Molekulargewicht beträgt etwa 130 000,

h) Untereinheiten:

sie besteht aus zwei identischen Untereinheiten mit einem Molekulargewicht von jeweils etwa 65 000,

i) Isoelektrischer Punkt:

der isoelektrische Punkt liegt bei pH 4,2,

j) Aminosäuresequenz:

die ersten 48 N-terminalen Aminosäuren sind:

```
Gly-Ser-Arg-Glu-Ile-Leu-Asp-Phe-Lys-Asp-      10

Leu-Ser-Ser-Pro-Ser-Ala-Pro-Ala-Glu-Leu-      20

Val-Ala-Asn-Ala-Ala-Phe-Leu-Glu-Pro-Ala-      30

Gly-His-Ala-Ala-Ala-His-Glu-Pro-Phe-Asn-      40

Gly-Gln-Ile-Thr-Leu-Gly-Glu-Thr-              48
```

Die Erfindung betrifft außerdem ein Verfahren zur Gewinnung dieser L-Carnitin-Amidase, welches dadurch gekennzeichnet ist, daß man den Stamm DSM 6230 in einem wäßrigen Nährmedium, das Mineralsalze, einen der oben genannten Induktoren und eine Quelle für Kohlenstoff und Stickstoff enthält, bei einer Temperatur von 20°C bis 30°C und einem Ausgangs-pH-Wert zwischen 6,5 und 8,5 aerob kultiviert, die Zellmasse abtrennt und das Enzym aus den Zellen isoliert.

Sie betrifft schließlich die Verwendung des Mikroorganismus DSM 6230 oder der daraus erhältlichen L-Carnitin-Amidase für die enzymatische Umsetzung von L- und/oder DL-Carnitinamid zu L-Carnitin.

Der Mikroorganismenstamm DSM 6230 wurde am 01.Oktober 1990 bei der Deutschen Sammlung von Mikroorganismen und Zellkulturen GmbH hinterlegt. Er wächst in Stäbchen mit einer Länge von 1,5 bis 2,5 $\mu$m und einer Breite von 0,5 bis 0,7 $\mu$m, zeigt keine Gram-Reaktion, durch 3 % KOH oder Aminopeptidase (Cerny) wird Lyse bewirkt. Der Stamm bildet keine Sporen und enthält eine Oxidase und eine Katalase. Er wächst nicht anaerob, aerob bei 37°C, aber nicht bei 41°C und nicht bei einem pH von 5,6, er wächst auf Mac-Conkey-Agar, aber nicht auf SS-Agar oder Cetrimid-Agar. Er enthält keine nicht diffundierenden oder diffundierenden und fluoreszierenden Pigmente und kein Pyocyanin. Er bildet beim OF-Test aus Glucose weder aerob noch anaerob Säuren und entwickelt aus Glucose kein Gas. Beim ASS-Test bildet er Säuren aus Glucose und Xylose, aber nicht aus Fructose. Er bildet keine ADH, ODC, LDC, ONPG, VP und kein Indol. Er reduziert $NO_3$ nicht zu $NO_2$ und bewirkt keine Denitrifikation. Er bildet eine Phenylalanindesaminase, kein Levan aus Saccharose, keine Lecithinase und keine Urease.

Er hydrolysiert nicht Stärke, Gelatine, Casein, DNA, Tween 80 oder Äsculin und baut Tyrosin nicht ab. Als Substrate verwertet er Acetat, Malat, Phenylacetat und Fructose, aber nicht Adipat, Citrat, Glycolat, Lactat, Laevulinat, Malonat, Suberat, L-Arabinose, Glucose, Mannose, Maltose. Xylose, Mannitol. Gluconat, 2-Ketogluconat, N-Acetylglucosamin, L-Serin, L-Histidin und Hydroxybutyrat. Die Hauptchinonkomponente ist das Ubichinon 10.

Der Mikroorganismenstamm DSM 6230 ist keiner bisher beschriebenen Spezies eindeutig zuzuordnen. Aufgrund seines zellulären Fettsäuremusters und der Chinonbestandteile ist dieses Bakterium aber der α-Untergruppe der Purpurbakterien zuzuordnen und steht somit zum Beispiel den Gattungen Agrobacterium und Sphingomonas nahe. Zu den authentischen Stämmen der Gattung Pseudomonas kann aufgrund der Chinonzusammensetzung und der zellulären Fettsäuren eine eindeutige Abgrenzung erfolgen.

Die wichtigsten morphologischen, physiologischen und biochemischen Eigenschaften sind in der nachfolgenden Tabelle zusammengestellt:

| | | | |
|---|---|---|---|
| Zellform | Stäbchen | NO₂ aus NO₃ | - |
| Breite µm | 0,5-0,7 | | |
| Länge µm | 1,5-2,5 | Denitrifikation | - |
| Gram-Reaktion | - | Phenylalanindesaminase | + |
| Lyse durch 3 % KOH | + | | |
| Aminopeptidase (Cerny) | + | Levan aus Saccharose | - |
| Sporen | - | Lecithinase | - |
| Oxidase | + | Urease | - |
| Catalase | + | Hydrolyse von | |
| | | Stärke | - |
| Wachstum | | Gelatine | - |
| anaerob | - | Casein | - |
| 37/41 °C | +/- | DNA | - |
| pH 5,6 | - | Tween 80 | - |
| Mac-Conkey-Agar | + | Äsculin | - |
| SS-Agar | - | | |
| Cetrimid-Agar | - | Tyrosin-Abbau | - |
| Pigmente | - | Substratverwertung | |
| nicht diffundierend | - | Acetat | + |
| diffundierend | - | Adipat | - |
| fluoreszierend | - | Citrat | - |
| Pyocyanin | - | Glycolat | - |
| | | Lactat | - |
| Säure aus (OF-Test) | | Lävulinat | - |
| Clucose aerob | - | Malat | + |
| Glucose anaerob | - | Malonat | - |
| | | Phenylacetat | + |
| Gas aus Glucose | - | Suberat | - |
| | | L-Arabinose | - |
| Säure aus (ASS) | | Fructose | + |
| Glucose | + | Glucose | - |
| Fructose | - | Mannose | - |
| Xylose | + | Maltose | - |
| | | Xylose | - |
| ADH | - | Mannitol | - |
| | | Gluconat | - |
| ODC | - | 2-Ketogluconat | - |
| | | N-Acetylglucosamin | - |
| LDC | - | L-Serin | - |
| | | L-Histidin | - |
| ONPG | - | Hydroxybutyrat | - |
| VP | - | Hauptchinonkomponente: | |
| | | Ubichinon 10 | |
| Indol | - | | |

L-Carnitin (3-Hydroxy-4-trimethylaminobutyrat) ist die ausschließlich physiologisch wirksame Form von Carnitin. Es ist ubiquitär im Tierreich verbreite und auch bei Pflanzen nachgewiesen worden. D-Carnitin wurde bisher noch nie in tierischen Geweben nachgewiesen. L-Crnitin transportiert langkettige, aktivierte Fettsäuren durch die innere Mitochondrienmembran in die mitochondriale Matrix, wo die $\beta$-Oxidation der Fettsäuren stattfindet. Kürzerkettige Fettsäuren können die innere Mitochondrienmembran frei passieren. Carnitinmangel beeinträchtigt also die Oxidation langkettiger Fettsäuren. Carnitinmangelerscheinungen werden durch Substitutionstherapie mit L-Carnitin behandelt. Da die notwendigen L-Carnitinmengen nicht immer zur Verfügung standen, wurde oft das racemische DL-Carnitin verabreicht. DL-Carnitin wurde Europa als Appetitstimulans, als Zusatzstoff für Sportlernahrung, zur Behandlung von Herzkrankheiten und Fettsucht eingesetzt. In letzter Zeit besteht jedoch die Tendenz, ausschließlich L-Carnitin zu verabreichen, da man festgestellt hat, daß D-Carnitin ein kompetetiver Inhibitor von Carnitin verbundenen Enzymen ist und infolgedessen unerwünschte Nebenwirkungen verursachen kann.

Seit der Entdeckung des L-Carnitins im Muskel gewinnt man L-Carnitin in aufwendigen Isolierungs- und Reinigungsverfahren aus tierischem Material. In den 50er Jahren wurden mehrstufige chemische Synthesen ausgearbeitet, die aber alle zum DL-Carnitin führen. Zur Isolierung des L-Isomeren wrden bis heute Verfahren benutzt, die auf der Racematspaltung mittels fraktionierter Kristallisation unter Anwendung optisch aktiver Säuren beruhen. Auch sind zahlreiche biochemische Verfahren zur Herstellung von L-Carnitin beschrieben: Beispielsweise die Hydroxylierung von $\gamma$-Butyrobetain, die Reduktion von 3-Dehydrocarnitin, die Umwandlung von Crotonobetain, die Hydrolyse von DL-O-Acylcarnitin mit einer Esterase oder die Hydrolyse von DL-Carnitinnitril. Für die industrielle Anwendung besitzen alle diese Verfahren Nachteile, da entweder die Ausgangsmaterialien teuer sind, die zur Anwendung kommenden Enzyme instabil sind oder über eine unzureichende Stereoselektivität oder Aktivität verfügen oder der Einsatz von teuren Coenzymen erforderlich ist.

Die vorliegende Erfindung beschreibt die Gewinnung von L-Carnitin aus DL- und/oder L-Carnitinamid. DL-Carnitinamid ist eine stabile Verbindung, die relativ kostengünstig ausgehend von Epichlorhydrin hergestellt werden kann.

Es gibt eine Veröffentlichung, wo die Hydrolyse von DL- und/oder L-Carnitinamid unter Einwirkung von Carnitinamid-Hydrolasen beschrieben ist (DOS 37 28 321). Die Enzyme werden produziert von Pseudomonaden, denen kein Artname zugeteilt ist. Alle diese Pseudomonaden verfügen über 2 Carnitinamid-Hydrolasen, ein L-spezifisches Enzym, dessen Substrat L-Carnitinamid ist und ein D-spezifisches Enzym, das D-Carnitinamid umsetzt. Das Wirkungsverhältnis beider Enzyme kann je nach Stamm und Wachstumsbedingungen beträchtlich variieren. Näher beschrieben ist nur die L-Carnitinamid-Hydrolase.

In der folgenden Tabelle sollen die Eigenschaften dieser L-Carnitinamid-Hydrolase denen der in dieser Erfindung beschriebenen L-Carnitin-Amidase gegenübergestellt werden.

| | L-Carnitinamid-Hydrolase | | L-Carnitin-Amidase | |
|---|---|---|---|---|
| Induzierbarkeit: | + | | + | |
| | | | | |
| Induktoren: | | | | |
| Carnitin | + | | + | |
| Carnitinamid | + | | + | |
| γ-Butyrobetain | + | | + | |
| | | | | |
| Substratspektrum: | | | | |
| D-Carnitinamid | − | | − | |
| Acetamid | − | | − | |
| Butyramid | − | | − | |
| Benzamid | − | | − | |
| | | | | |
| Inhibitoren: | (1 mM) | (10 mM) | (1 mM) | (10 mM) |
| $Ag^{2+}$ | + | + | + | n.b. |
| $Cu^{2+}$ | − | k.A. | + | + |
| $Mn^{2+}$ | − | k.A. | + | + |
| $Mg^{2+}$ | − | k.A. | + | + |
| $Zn^{2+}$ | − | k.A. | + | n.b. |
| $Co^{2+}$ | − | k.A. | + | + |
| $Fe^{2+}$ | − | + | + | + |
| 2-Mercaptoethanol | − | − | − | − |
| EDTA | − | − | + | + |
| | | | | |
| pH-Optimum: | pH 6 – pH 7 | | pH 7 – pH 9,5 | |
| stabiler pH-Bereich | pH 5 – pH 8 | | pH 5 – pH 9,5 | |
| Molekulargewicht: | ca. 36000 | | ca. 130000 | |
| Untereinheiten: | k.A. | | 2 | |

-------------

k.A. = keine Angaben

n.b. = nicht bestimmt

Beide Enzyme zeigen Parallelen was die Induzierbarkeit, die Induktoren, das Substratspektrum, die Inhibierung durch Silberionen (1 mM) und durch Ferroionen (10 mM) und die fehlende Inhibierung durch 2-Mercaptoethanol anbelangt.

Bei den anderen aufgeführten Eigenschaften gibt es keine Übereinstimmung. Spätestens beim Vergleich der Molekulargewichte wird deutlich, daß es sich bei der L-Carnitinamid-Hydrolase und bei der L-Carnitin-Amidase, die in dieser Erfindung beschrieben wird, um zwei vollkommen verschiedene Enzyme handelt.

Die Erfindung soll durch die nachstehenden Beispiele näher erläutert werden:

Beispiel 1:

Suche nach Carnitin-Amidasen

In einem umfangreichen Screening wurden Boden-, Gewässer- und Klärwerksproben von insgesamt 267 Standorten untersucht. Gearbeitet wurde mit Direct-Plating und Anreicherungskulturen, mit Voll- und Minimalmedien, mit Minimalmedien und zusätzlichen Kohlenstoffquellen und mit verschiedenen Säureamiden als Induktoren und/oder Kohlenstoff- und/oder Stickstoffquellen. Dazu wurden die Proben mit einer 0,9 %igen Kochsalzlösung aufgeschlämmt beziehungsweise verdünnt und zur guten Durchmischung beziehungsweise Ausspülung für 4-6 Stunden geschüttelt. Danach wurden die Suspensionen bei niedriger Umdrehungszahl kurz anzentrifugiert, um die festen Bestandteile zu sedimentieren. Aliquots der Überstände wurden entweder nach geeigneter Verdünnung auf Platten mit festen Medien ausplattiert, oder in 100 ml Erlenmeyerkolben gegeben, die 20 ml Flüssigmedium enthielten. Die Platten wurden für 3-7 Tage im Brutschrank bebrütet, bevor von ihnen Einzelkolonien isoliert und in Reinkultur gebracht wurden.

Die Flüssigkulturen wurden in einem Rundschüttler bei 30°C und 120 Upm für 3-7 Tage bebrütet. Nach dieser Zeit wurden Aliquots aus den Kulturflüssigkeiten entnommen, und mit ihnen neue 20 ml Flüssigkulturen angeimpft, die unter den gleichen Bedingungen bebrütet wurden. Dieser Vorgang wurde ca. fünfmal wiederholt, bevor Proben der Kulturflüssigkeiten nach geeigneter Verdünnung auf Platten mit festem Medium ausplattiert wurden. Die Platten wurden 3-7 Tage bei 30°C im Brutschrank bebrütet, bevor von ihnen Einzelkulturen isoliert und in Reinkultur gebracht wurden.

Medienzusammensetzung:

| Minimalmedium: | | |
|---|---|---|
| $K_2HPO_4$ | 2,50 | g |
| $KH_2PO_4$ | 1,95 | g |
| $NaCl$ | 1,00 | g |
| $CaCl_2 \times 2\ H_2O$ | 0,05 | g |
| $MgSO_4 \times 7\ H_2O$ | 0,30 | g |
| Hefeextrakt | 0,50 | g |
| DL-Carnitinamid | 5,00 | g |
| Spurensalzlösung | 0,80 | ml |

aufgefüllt mit destilliertem Wasser
auf 1,0 l pH 7,2
Platten mit 1,8 % Agar

| Spurensalzlösung: | | |
|---|---|---|
| $H_3BO_3$ | 75,0 | mg |
| $MnCl_2 \times 4\ H_2O$ | 50,0 | mg |
| $ZnCl_2$ | 187,5 | mg |
| $CuSO_4 \times 5H_2O$ | 50,0 | mg |
| $FeCl_3 \times 6H_2O$ | 625,0 | mg |
| $(NH_4)_8Mo_7O_{24} \times H_2O$ | 25,0 | mg |
| $CoSO_4 \times 7H_2O$ | 37,5 | mg |

aufgefüllt mit destilliertem Wasser
auf 0,2 l

```
Minimalmedium + zusätzliche Kohlenstoffquellen:

                        Minimalmedium +

                        Glycerin                2,50 ml

                        und/oder Äpfelsäure      2,50 g


Minimalmedium + zusätzliche Säureamide:

                        Minimalmedium mit 2,5 g

                        DL-Carnitinamid + Acetamid bzw.

                        Propionamid             2,50 g


Vollmedium:             Minimalmedium +

                        Casein                   0,50 g

                        Pepton                   0,50 g

                        Fleischextrakt           0,50 g

                        Malzeexrakt              0,50 g

                        Glycerin                 0,50 g
```

$CaCl_2$, $MgSO_4$, DL-Carnitinamid, Acetamid und Propionamid wurden den Kulturflüssigkeiten nach dem Autoklavieren unter sterilen Bedingungen zugegeben.

Über das gesamte Screening hinweg wurden 1367 Stämme isoliert. Diese Stämme wurden in 100 ml Erlemeyerkolben, die 20 ml des jeweiligen Anreicherungsmediums enthielten in einem Rundschüttler bei 30°C und 120 Upm bebrütet. Als Indiz für das Zellwachstum wurde die optische Dichte bei 660 nm herangezogen.

Nach 3 Tagen wurde der Inhalt der Schüttelkolben zentrifugiert (10 min 10000 Upm in einer Kühlzentrifuge) und die sedimentierten Zellen in 100 mM Kaliumphosphatpuffer pH 7.5 aufgenommen (es wurde eine ca. 20 % ige Mikroorganismensuspension hergestellt).

Die Mikroorganismen in dieser Suspension müssen in üblicher Weise aufgeschlossen werden (z.B. Schütteln mit feinen Glasperlen oder Ultraschallbehandlung). Der Zellaufschluß erfolgte dann durch Naßvermahlung mit Glasperlen (Durchmesser 0,3 mm; 2 g Perlen pro 1 ml Zellsuspension). Durch Zentrifugation in einer Kühlzentrifuge (5 min 12000 Upm) wurden die Glasperlen und die groben Zelltrümmer abzentrifugiert. Der klare Überstand wurde als Rohextrakt bezeichnet und für weitere Experimente eingesetzt.

Die Bestimmung der Proteinkonzentration wurde nach der Methode von Bradford durchgeführt.

Bei der Umsetzung von Carnitinamid zum Carnitin werden Carnitin und Ammoniak im gleichen molaren Verhältnis gebildet.

$$Carnitinamid + H_2O \xrightarrow{\text{Carnitin-Amidase}} Carnitin + NH_3$$

Ansätze für die Bestimmung von Enzymaktivitäten sind also:
- Messung der Abnahme des Carnitinamids
- Messung des gebildeten Carnitins
- Messung des gebildeten Ammoniaks

Die Ansätze zum Aktivitätstest beinhalteten:
400 $\mu$l Kaliumphosphatpuffer 0,1 M, pH 7,5
50 $\mu$l Substrat in 0,1 M Kaliumphosphatpuffer, pH 7,5
50 $\mu$l Enzymlösung (Roheextrakt)

Als Substrate wurden eingesetzt:

DL-Carnitinamid Endkonzentration im Test 100 mM

D-Carnitinamid Endkonzentration im Test 50 mM

L-Carnitinamid Endkonzentration im Test 50 mM

Der Enzymtest wurde in 1,5 ml Eppendorfgefäßen bei einer Temperatur von 30°C durchgeführt. Die Reaktion wurde durch Zugabe von Enzymlösung gestartet und durch Ansäuern gestoppt. Das gefällte Protein wurde abzentrifugiert (Eppendorfzentrifuge 5 min 12500 Upm) und der Überstand untersucht. Zur Bestimmung des Leerwertes lief stets eine Nullprobe für das Substrat und die Enzymlösung mit.

Die Definition der Enzymeinheit ist 1 mU = 1 nM umgesetztes Substrat pro Minute.

Der Nachweis von Carnitin und Carnitinamid kann über Dünnschichtchromatographie erfolgen. Als Adsorbens dienten Kieselgelplatten der Firma Merck, auf die jeweils 3 $\mu$l der zu untersuchenden Flüssigkeiten aufgetragen wurden. Detektiert wurde Carnitin und Carnitinamid mit Joddampf.

Die Zusammensetzung der Laufmittel und die $R_F$-Werte sind der folgenden Tabelle zu entnehmen.

| Laufmittel | Substanz | $R_F$-Wert |
|---|---|---|
| Chloroform/Methanol/Wasser/ Ameisensäure konz./Ammoniak konz. 50/55/10/5/7,5 | Carnitin | 0,44 |
| | Carnitinamid | 0,51 |
| Chloroform/Methanol/Wasser/ Ameisensäure konz. 50/50/17,25/0,425 | Carnitin | 0,35 |
| | Carnitinamid | 0,45 |

Bestimmt werden können Carnitin und Carnitinamid über die HPLC

System:

| | |
|---|---|
| - Stationäre Phase: | $NH_3$ |
| - Säule - Säulennummer: | BK 3/88 |
| - Säulendurchmesser innen: | 5 mm |
| - Temperatur: | 45°C |
| - Mobile Phase A: | 670 ml Acetonitril f. Chr. |
| - Mobile Phase B: | 330 ml 0.05 M $(NH_4)_2 HPO_4$ |
| - Flußrate: | 0,7 ml/min |
| - Wellenlänge: | 205 nm |
| - Injektionsvolumen | 20 $\mu$l |

L-Carnitin kann enzymatisch mit der Carnitin-Acetyltransferase bestimmt werden. Der Ammoniak wurde zum einen photometrisch als Indophenol und zum anderen enzymatisch mit Hilfe der Glutamatdehydrogenase bestimmt.

Von den 1387 isolierten Stämmen besitzen 65 Amidaseaktivität. Die Enzymaktivitäten reichen von einigen mU bis zu mehreren U. Aus den aktiven Stämmen wurde einer ausgewählt, um ihn bei der Deutschen Sammlung von Mikroorganismen und Zellkulturen (DSM) bestimmen zu lassen.

Der Rohextrakt dieses Stammes wurde mit enantiomerenreinem D- und L-Carnitinamid getestet:

| Aktivität D-Amid | (mU/ml) L-Amid | Aktivität D-Amid | (mU/mg) L-Amid | L-Amidaseaktivität (%) |
|---|---|---|---|---|
| 0 | 8624 | 0 | 1960 | 100 |

Beispiel 2:

Genauere Untersuchung der Amidaseaktivität

Der Rohextrakt wurde erneut auf D- und L-Carnitinamid getestet und der Enzymtest nach 10 min (es ist noch ausreichend L-Carnitinamid im Testansatz vorhanden) und nach 5 Stunden (das L-Carnitinamid ist weitgehend oder vollständig abreagiert) gestoppt.

Die L-Amidaseaktivität, bezogen auf die Gesamtamidaseaktivität, betrug nach 10 min 100 % und nach 5 Stunden immer noch über 99 %.

Dieser Versuch zeigt, daß der Rohextrakt des Stammes DSM 6230 selbst nach einer Inkubationszeit von 5 Stunden kaum meßbar D-Carnitin bildet. Dieser Stamm weist demnach neben einer hohen Enzymaktivität eine sehr hohe Selektivität der Amidasereaktion auf.

Beispiel 3:

Versuch zur Induktion der L-Carnitin-Amidase

In diesem Versuch wurden verschiedene Substanzen auf ihre Induktionswirkung untersucht. Gleichzeitig wurde das Wachstum auf diesen Verbindungen verfolgt. Als Medium diente ein Minimalmedium mit 1,0 g/l statt 0,5 g/l Hefeextrakt, dem jeweils 5 g/l der zu testenden Substanz zugefügt wurden. Die Kultur ohne Induktor enthielt 5 g/l Hefeextrakt. Alle Versuche wurden mit 20 ml Schüttelkulturen durchgeführt, die 36 h bei 30°C und 120 Upm in einer Rundschüttelmaschine bebrütet wurden. Angeimpft wurden diese Kulturen mit 1 % einer Vorkultur von Stamm DSM 6230. Für den Enzmtest wurde L-Carnitinamid als Substrat eingesetzt.

Die Ergebnisse dieses Versuchs zeigt folgende Tabelle:

| Induktor | $OD_{660}$ | Aktivität (mU/ml) | Aktivität (mU/mg) | mU/mg (%) |
|---|---|---|---|---|
| L-Carnitinamid | 3,5 | 8530 | 1706 | 100 |
| ohne | 2,8 | 129 | 39 | 2 |
| D-Carnitinamid | 0,8 | 497 | 166 | 10 |
| DL-Carnitinamid | 2,7 | 8867 | 1673 | 98 |
| DL-Carnitin | 2,5 | 5773 | 1255 | 74 |
| $\gamma$-Butyrobetain | 0,7 | 963 | 566 | 33 |
| Dehydrocarnitin | 0,8 | 837 | 279 | 16 |
| Glycinbetain | 2,9 | 183 | 33 | 2 |
| D-Carnitinnitril | 0,8 | 206 | 90 | 5 |
| L-Carnitinnitril | 0,8 | 183 | 65 | 4 |
| DL-Carnitinnitril | 0,8 | 221 | 88 | 5 |
| D-Leucinamid | 0,8 | 170 | 81 | 5 |
| L-Leucinamid | 2,5 | 34 | 9 | <1 |
| L-Prolinamid | 1,0 | 250 | 54 | 3 |
| L-Phenylalaninamid | 0.6 | 172 | 72 | 4 |
| L-Valinamid | 0,9 | 32 | 12 | <1 |
| Glycinamid | 1,0 | 180 | 43 | 3 |
| Acetamid | 1,0 | 0 | 0 | 0 |
| Propionamid | 1,1 | 129 | 29 | 2 |
| Butyramid | 0,9 | 54 | 18 | 1 |
| Isobutyramid | 1,1 | 193 | 41 | 2 |
| Milchsäureamid | 0,8 | 55 | 28 | 2 |
| Acrylamid | 0,8 | 170 | 136 | 8 |
| Methacrylamid | 0,8 | 237 | 76 | 4 |
| Benzamid | 0,3 | 0 | 0 | 0 |
| Nikotinamid | 0,7 | 55 | 28 | 2 |

Der Versuch zeigt eindeutig, daß die L-Carnitin-Amidase ein induzierbares Enzym ist. Induktoren sind: L-Carnitinamid, L-Carnitin, $\gamma$-Butyrobetain und Dehydrocarnitin. Der beste der getesteten Induktoren ist L-Carnitinamid.

Gutes Wachstum zeigt der Stamm auf L-Leucinamid, L-Carnitinamid, DL-Carnitinamid, DL-Carnitin und Glycinbetain. L-Leucinamid und Glycinbetain sind lediglich gute Wachstumssubstrate, die L-Carnitin-Amidase wird durch sie nicht induziert. $\gamma$-Butyrobetain und Dehydrocarnitin hingegen induzieren das Enzym, können aber nicht, oder sehr schlecht verstoffwechselt werden.

Beispiel 4:

Bestimmung der optimalen Induktorkonzentration

In einem weiteren Versuch wurde die Induktion der L-Carnitin-Amidase bei unterschiedlichen Konzentrationen von DL-Carnitinamid im Kulturmedium untersucht. Gleichzeitig wurde das Wachstum der Bakterien bei diesen Carnitinamidkonzentrationen verfolgt. Die Kulturbedingungen entsprachen denen aus Beispiel 3. Die Ergebnisse dieses Versuchs zeigt folgende Tabelle:

| DL-Carnitinamidkonzentration (g/l) | $OD_{660nm}$ | Amidaseaktivität (U/ml) |
|---|---|---|
| 0,5 | 1,1 | 0,7 |
| 1,0 | 1,3 | 2,4 |
| 2,0 | 1,7 | 6,1 |
| 3,0 | 2,0 | 7,3 |
| 5,0 | 2,8 | 8,9 |
| 7,5 | 4,0 | 10,6 |
| 10,0 | 3,9 | 8,6 |
| 15,0 | 3,7 | 7,3 |
| 20,0 | 3,6 | 7,1 |
| 25,0 | 3,5 | 6,7 |

Dieser Versuch zeigt, daß die optimale DL-Carnitinamidkonzentration, sowohl für das Wachstum der Bakterien als auch für die Amidaseaktivität unter den oben genannten Kulturbedingungen 7,4 g/l beträgt.

Beispiel 5:

L-Carnitin-Amidasefermentation im 8 1 Maßstab Verwendet wurde ein Bioreaktor, der folgendermaßen ausgerüstet war:
- Rührwerk
- Drehzahlregelung
- Temperaturregelkreis mit Thermofühler
- Sterilfilter für Zu- und Abluft
- Abluftkühlung
- pH-Elektrode
- Antischaumsonde
- Säure-, Lauge und Antischaumvorratsgefäße
- Sauerstoffelektrode

Das Arbeitsvolumen betrug 8 1, der pH-Wert des Fermentationsmediums 7,2 und die Fermentationstemperatur 30°C. Das Medium war ein Minimalmedium (siehe Beispiel 1) mit 1 g/l Hefeextrakt und 7,5 g/l DL-Carnitinamid. Nach der Sterilisation wurde der Fermenter mit 200 ml einer Vorkultur von Stamm DSM 6230, die 24 h bei 120 Upm und 30°C in einem Rundschüttler bebrütet worden war, angeimpft.

Nach 17 h wurde die Fermentation beendet. Die $OD_{660}$ der Kulturflüssigkeit betrug zu diesem Zeitpunkt 3,2. Es konnten 63 g feuchte Zellen geerntet werden. Mit 100 mM Kaliumphosphatpuffer pH 7,5 wurde eine 20 %ige Zellsuspension hergestellt (Endvolumen 315 ml). Aufgeschlossen wurden die Zellen durch Naßvermahlung mit Glasperlen (Durchmesser 0,3 mm). Die Volumenaktivität pro ml Rohextrakt betrug 9590 mU und die spezifische Aktivität 1314 mU. Aus 8 1 Fermentationsmedium konnten somit 3021 Units L-Carnitin-Amidase gewonnen werden. In dem Rohextrakt konnte keine D-Carnitin-Amidaseaktivität gemessen werden.

Beispiel 6:

Aufreinigung der L-Carnitin-Amidase

Die L-Carnitin-Amidase ließ sich aus dem Rohextrakt nach Fällung der Nukleinsäuren mit Polyethylenimin (0,1 %) über zwei Ionenaustauscher ("Fractogel EMDTMAE-650 (S)" der Firma Merck und "Mono- Q" der Firma Pharmacia) bis zur vollständigen Reinheit aufreinigen. Die spezifischen Aktivitäten betrugen im Rohextrakt 1.31 U pro mg Protein und 328 U pro mg Mono-Q-gereinigtem Enzym. Der Anreicherungsfaktor für das Enzym betrug also 250.

Beispiel 7:

Bestimmung des isoelektrischen Punktes

Die Bestimmung des isoelektrischen Punktes der L-Carnitin-Amidase wurde nach Pharmacia Phast-System Methode 100 durchgeführt.
Er liegt bei pH 4,2.

Beispiel 8:

Bestimmung des Molekulargewichtes und der Anzahl der Untereinheiten

Das Molekulargewicht des nativen Enzyms wurde durch Gelfiltration an Sephacryl S-200 HR ermittelt. Die an eine FPLC-System gekoppelte Säule (1,6 × 69,6 cm) wurde mit einer Durchflußrate von 1ml/min betrieben. Als Eichproteine dienten Cytochrom C, Myoglobin (Wal), Myoglobin (Pferd), Carboxyanhydrase B. Ovalbumin, BSA, R-Oxinirilase (Hirse), Aldolase (Monomer) und Aldolase (Dimer) (Kaninchenmuskel). Das Molekulargewicht der L-Carnitin-Amidase beträgt 125000 ± 5000.
In der Gelelektrophorese in Gegenwart von Natriumdodecylsulfat (SDS) wurde für das denaturierte Enzym ein Molekulargewicht von 65700 ermittelt. Demnach besteht die Amidase aus zwei identischen Untereinheiten. Als Eichproteine wurden $\alpha_2$-Macroglobulin (Pferd) nicht reduziert und reduziert, Phosphorylase b (Kaninchenmuskel), BSA, Glutamat-Dehydrogenase (Rinderleber), Lactat-Dehydrogenase (Schweineleber) und der Trypsin-Inhibitor aus der Sojabohne verwendet.

Beispiel 9:

pH-Abhängigkeit der L-Carnitin-Amidase

a) Abhängigkeit der Reaktionsgeschwindigkeit vom pH-Wert

Zur Bestimmung des pH-Optimums wurde der Enzymtest bei unterschiedlichen pH-Werten durchgeführt. Dabei wurden folgende Puffer verwendet: Citrat von pH 3 - pH 6,5, Kaliumphosphat von pH 6,5 - pH 8,5, Tris/HCl von pH 8,5 - pH 9,5 und Bicarbonat von pH 9,5 - pH 11. Das Substrat wurde vorher auf den entsprechenden pH-Wert eingestellt.
Enzymaktivität ist vorhanden von pH 4,5 - pH 11. Gute Umsätze werden erreicht im Bereich von pH 7 - pH 9,5. Die höchste Reaktionsgeschwindigkeit wird bei pH 8,5 in Tris/HCl-Puffer erzielt.

b) Einfluß des pH-Wertes auf die Enzymstabilität

Zur Bestimmung der Enzymstabilität in Abhängigkeit vom pH-Wert wurde Enzymlösung mit verschiedenen Puffern unterschiedlicher pH-Werte 10-fach verdünnt (siehe a) und bei 30°C für 30 min inkubiert. Dann wurden diese Ansätze nach Abkühlen (2 min auf Eis) 5 min bei 13000 Upm zentrifugiert. Jeweils 50 µl dieser Überstände wurden als Enzymlösung im üblichen Enzymtest, der jedoch diesmal in 200 mM Kaliumphosphatpuffer durchgeführt wurde, eingesetzt. Das Enzym zeigte gute Stabilität im Bereich von pH 5,0 bis pH 9,5. Die beste Stabilität wird zwischen pH 7,5 und pH 8,5 erzielt.

Beispiel 10:

Einfluß von Zusatzstoffen auf die L-Carnitin-Amidaseaktivität

Um den Einfluß verschiedener Zusatzstoffe auf die Enzymaktivität zu überprüfen, wurden diese den Testansätzen ohne Substrat in einer Endkonzentration von jeweils 1 mM und 10 mM zugegeben. Die Reaktionsansätze wurden zunächst für 45 min bei 30°C inkubiert, bevor die Reaktion durch Zugabe von Substratlösung gestartet wurde.
Die Ergebnisse dieser Versuche zeigt folgende Tabelle:

| Zusatzstoff | Enzymaktivität (%) | |
| --- | --- | --- |
| | 1 mM | 10 mM |
| ohne | 100 | |
| Bi- und Trivalente Kationen: | | |
| $MgCl_2$ | 96 | 88 |
| $CaCl_2$ | 103 | 90 |
| $BaCl_2$ | 102 | 105 |
| $MnCl_2$ | 89 | 76 |
| $ZnCl_2$ | 6 | n.b. |
| $FeCl_2$ | 86 | 57 |
| $FeCl_3$ | 87 | 46 |
| $CoCl_2$ | 90 | 76 |
| $NiCl_2$ | 80 | 79 |
| $CuCl_2$ | 70 | 54 |
| Komplexbildner: | | |
| Titriplex III (EDTA) | 93 | 73 |
| Phenanthrolin | 89 | 82 |
| Diethylmalonsäure | 92 | 68 |
| $NaN_3$ | 85 | 82 |

reduzierende Agenzien:

| | | |
|---|---|---|
| 2-Mercaptoethanol | 100 | 101 |
| Red. Glutathion | 97 | 72 |
| Ethylmaleinimid | 100 | 99 |
| Dithiothreitol (DTT) | 94 | 101 |
| Dithioerythrit (DTE) | 98 | 99 |

SH-Gruppenreagenzien:

| | | |
|---|---|---|
| para-Hydroxymercuribenzoat (pOHMB) | 51 | n.b. |
| Jodacetat | 97 | 72 |
| $HgCl_2$ | <1 | n.b. |
| $AgNO_3$ | 65 | n.b. |
| KCN | 85 | 88 |

Inhibitoren von PLP-Enzymen:

| | | |
|---|---|---|
| Cycloserin | 96 | 97 |

Inhibitoren von Proteasen:

| | | |
|---|---|---|
| Neostigminbromid | 100 | 94 |
| para-Aminobenzamidindihydrochlorid (pABA) | 88 | 99 |
| Phenylmethansulfonylfluorid (PMSF) | 23 | 2 |
| Na-Tosyl-L-lysyl-chlormethanhydrochlorid (TLCK) | 84 | 73 |
| Dinitrodithiodibenzoesäure | 34 | n.b. |
| α-Ketoisocapronsäure | 92 | 90 |
| NaF | 88 | 78 |
| Acetaldehyd | 83 | 81 |

-------------------

n.b. = nicht bestimmt

Deutlich inhibierend wirken bereits bei einer Konzentration von 1 mM die zweiwertigen Metallionen $Zn^{2+}$ und $Cu^{2+}$, die SH-Gruppenreagenzien $Hg^{2+}$, $Ag^{2+}$ und p-Hydroxymercuribenzoat, ferner die Proteaseinhibitoren Phenylmethansulfonylfluorid und Dinitrodithiodibenzoesäure. Bei einer Konzentration von 10 mM bewirken $Mn^{2+}$, $Fe^{2+}$, $Fe^{3+}$, $Co^{2+}$, EDTA, Diethylmalonsäure, red. Glutathion, Jodacetat, TLCK und NaF eine Verminderung der Enzymaktivität.

Beispiel 11:

Substratspektrum der L-Carnitin-Amidase

14

Der Enzymtest wurde durchgeführt bei 30°C in 100 mM Kaliumphosphatpuffer pH 8. Die Substratkonzentration betrug 50 mM.

Die Ergebnisse dieses Versuchs zeigt folgende Tabelle:

| Substrat | rel. Aktivität (%) | Substrat | rel. Aktivität (%) |
|---|---|---|---|
| L-Carnitinamid | 100 | Aminosäureamide: | |
| D-Carnitinamid | 0 | L-Pro-NH$_2$ | 0 |
| D-Carnitinnitril | 0 | L-Ser-NH$_2$ | 0 |
| L-Carnitinnitril | 0 | L-Thr-NH$_2$ | 0 |
| Acetamid | 0 | D-Phe-NH$_2$ | 0 |
| Propionamid | 0 | L-Phe-NH$_2$ | 0 |
| Butyramid | 0 | L-Tyr-NH$_2$ | 0 |
| Isobutyramid | 0 | L-Trp-NH$_2$ | 0 |
| Milchsäureamid | 0 | L-Arg-NH$_2$ | 0 |
| Acrylamid | 0 | L-Arg-NH$_2$ | 0 |
| Methacrylamid | 0 | L-His-NH$_2$ | 0 |
| Benzamid | 0 | L-Asn-NH$_2$ | 0 |
| | | L-Met-NH$_2$ | 0 |
| Aminosäureamide: | | | |
| Gly-NH$_2$ | 0 | Dipeptidamide: | |
| L-Val-NH$_2$ | 0 | L-Val-L-Phe-NH$_2$ | 0 |
| D-Leu-NH$_2$ | 0 | L-Asp-L-Phe-NH$_2$ | 0 |
| L-Leu-NH$_2$ | 0 | L-Ala-L-Phe-NH$_2$ | 0 |
| L-Ile-NH$_2$ | 0 | L-Tyr-Gly-NH$_2$ | 0 |
| L-Ala-NH$_2$ | 0 | Gly-Gly-NH$_2$ | 0 |

Die L-Carnitin-Amidase zeichnet sich durch eine sehr hohe Substratsspezifität aus. Außer L-Carnitinamid wurde keines der getesteten Amide umgesetzt. Dünnschichtchromatographische Untersuchungen zeigten, daß die Amidase auch keine proteolytische Aktivität auf die getesteten Dipeptide hat.

Beispiel 12:

Aminosäuresequenz der L-Carnitin-Amidase

Die Aminosäuresequenz der ersten 48 N-terminalen Aminosäuren wurde auf einem Proteinseqenzator bestimmt. Dazu wurde Mono-Q-gereinigtes und durch Ultrafiltration aufkonzentriertes Enzym, gelöst in 10 mM Kaliumphosphatpuffer pH 7,5, aufgegeben.

Die Aminosäuresequenz lautet wie folgt:

```
Gly-Ser-Arg-Glu-Ile-Leu-Asp-Phe-Lys-Asp-              10

Leu-Ser-Ser-Pro-Ser-Ala-Pro-Ala-Glu-Leu-             20

Val-Ala-Asn-Ala-Ala-Phe-Leu-Glu-Pro-Ala-            30

Gly-His-Ala-Ala-Ala-His-Glu-Pro-Phe-Asn-            40

Gly-Gln-Ile-Thr-Leu-Gly-Glu-Thr-                    48
```

Beispiel 13:

Umsatz mit ganzen Zellen

Der Umsatz mit ganzen Zellen wurde untersucht, indem mit einer 20 %-igen Zellsuspension des Stammes DSM 6230 der übliche Enzymtest durchgeführt wurde.
Ergebnis: L-Carnitinamid wird durch ganze Zellen mit 64 % höherer Aktivität umgesetzt als durch eine aufgeschlossene Zellsuspension gleicher Konzentration. D-Carnitinamid wird nicht umgesetzt.
Es ist also zu erwarten, daß die Umsetzung von L-Carnitinamid zum L-Carnitin auch sehr gut mittels immobilisierter Mikroorganismen durchgeführt werden kann. Die Immobilisierung der Mikroorganismen durch Einbetten in ein die Mikroorganismen nicht denaturierendes Polymeres erfolgt nach Methoden, die dem Fachmann wohlbekannt sind. Geeignete Mikroorganismen-Immobilisate sind Immobilisate auf Alginat- oder Chitosan-Basis.

**Patentansprüche**

1. Mikroorganismus mit der Hinterlegungsnummer DSM 6230.

2. Mikrobiologisch hergestellte L-Carnitin-Amidase, gekennzeichnet durch die folgenden Eigenschaften:
   a) Reaktivität:
   sie hydrolisiert L-Carnitinamid zu L-Carnitin und Ammoniak,
   b) Substratspezifität:
   sie hydrolysiert nicht D-Carnitinamid, keine aliphatischen oder aromatischen Carbonsäureamide, keine Aminosäureamide und keine Dipeptidamide,
   c) Induktoren:
   sie ist ein induzierbares Enzym und wird induziert durch L-Carnitinamid, L-Carnitin, $\gamma$-Butyrobetain und Dehydrocarnitin,
   d) pH-Optimum:
   der optimale pH-Bereich liegt zwischen pH 7,0 und pH 9,5,
   e) pH-Stabilität:
   sie zeigt eine gute pH-Stabilität im pH-Bereich zwischen pH 5,0 und pH 9,5,
   f) Inhibitoren:
   inhibierend wirken bereits bei einer Konzentration von 1 mM $Zn^{2+}$, $Cu^{2+}$, $Hg^{2+}$ und $Ag^{2+}$, ferner p-Hydroxymercuribenzoat, Phenylmethansulfonylfluorid und Dinitrodithiobenzoesäure,
   g) Molekulargewicht:
   das Molekulargewicht beträgt etwa 130 000,
   h) Untereinheiten:
   sie besteht aus zwei identischen Untereinheiten mit einem Molekulargewicht von jeweils etwa 65 000,
   i) Isoelektrischer Punkt:
   der isoelektrische Punkt liegt bei pH 4,2,
   j) Aminosäuresequenz:
   die ersten 48 N-terminalen Aminosäuren sind:

16

```
Gly-Ser-Arg-Glu-Ile-Leu-Asp-Phe-Lys-Asp-          10
Leu-Ser-Ser-Pro-Ser-Ala-Pro-Ala-Glu-Leu-          20
Val-Ala-Asn-Ala-Ala-Phe-Leu-Glu-Pro-Ala-          30
Gly-His-Ala-Ala-Ala-His-Glu-Pro-Phe-Asn-          40
Gly-Gln-Ile-Thr-Leu-Gly-Glu-Thr-                   48
```

3. Verfahren zur Gewinnung der L-Carnitin-Amidase gemäß Anspruch 2, dadurch gekennzeichnet, daß man den Stamm DSM 6230 in einem wäßrigen Nährmedium, das Mineralsalze, einen der oben genannten Induktoren und eine Quelle für Kohlenstoff und Stickstoff enthält, bei einer Temperatur von 20°C bis 30°C und einem Ausgangs-pH-Wert zwischen 6,5 und 8,5 aerob kuliviert, die Zellmasse abtrennt und das Enzym aus den Zellen isoliert.

4. Verwendung des Mikroorganismus gemäß Anspruch 1 oder der L-Carnitin-Amidase gemäß Anspruch 2 für die enzymatische Umsetzung von DL- und/oder L-Carnitinamid zu L-Carnitin.

Europäisches
Patentamt

**EUROPÄISCHER RECHERCHENBERICHT**

Nummer der Anmeldung

EP 92 10 2729

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | FR-A-2 603 303 (CHUO KASEIHIN CO ET AL.) <br> * das ganze Dokument * | 1-4 | C12N9/80 <br> C12N1/20 <br> //(C12N9/80, <br> C12R1/01) |
| D | & DE-A-3 728 321 <br> --- | | |
| Y | PATENT ABSTRACTS OF JAPAN <br> vol. 13, no. 546 (C-661)6. Dezember 1989 <br> & JP-A-1 222 796 ( KYOWA HAKKO KOGYO KK ) 6. September 1989 <br> * Zusammenfassung * <br> --- | 1-4 | |
| A | PATENT ABSTRACTS OF JAPAN <br> vol. 013, no. 546 (C-661)6. Dezember 1989 <br> & JP-A-1 222 797 ( KYOWA HAKKO KOGYO CO LTD ) 6. September 1989 <br> * Zusammenfassung * <br> ----- | 1-4 | |
| | | | **RECHERCHIERTE SACHGEBIETE (Int. Cl.5 )** <br><br> C12N <br> C12P |

Der vorliegende Recherchenbericht wurde für alle Patentansprüche erstellt

| Recherchenort | Abschlußdatum der Recherche | Prüfer |
|---|---|---|
| BERLIN | 09 APRIL 1992 | GURDJIAN D. |